(19) Europäisches Patentamt
European Patent Office
Office européen des brevets

(11) **EP 0 528 938 B1**

(12) **EUROPEAN PATENT SPECIFICATION**

(45) Date of publication and mention
of the grant of the patent:
**02.10.1996 Bulletin 1996/40**

(21) Application number: **91909789.9**

(22) Date of filing: **16.05.1991**

(51) Int. Cl.$^6$: **A61B 5/00**

(86) International application number:
**PCT/GB91/00775**

(87) International publication number:
**WO 91/17697 (28.11.1991 Gazette 1991/27)**

(54) **NON-INVASIVE MEDICAL SENSOR**

NICHTINVASIVER MEDIZINISCHER SENSOR

DETECTEUR MEDICAL NON-INVASIF

(84) Designated Contracting States:
**AT BE CH DE DK ES FR GB GR IT LI LU NL SE**

(30) Priority: **17.05.1990 GB 9011131**

(43) Date of publication of application:
**03.03.1993 Bulletin 1993/09**

(73) Proprietor: **JOHNSON & JOHNSON
PROFESSIONAL PRODUCTS LIMITED
Bracknell, Berkshire RG12 2AT (GB)**

(72) Inventor: **EVANS, Peter Dilwyn
Cardiff CF3 7ET (GB)**

(74) Representative: **Mercer, Christopher Paul
Carpmaels & Ransford
43, Bloomsbury Square
London WC1A 2RA (GB)**

(56) References cited:
EP-A- 0 290 279          US-A- 4 223 680
US-A- 4 281 931          US-A- 4 889 116
US-A- 4 914 512

* **MEDICAL AND BIOLOGICAL ENGINEERING
AND COMPUTING. vol. 26, no. 3, May 1988,
STEVENAGE GB pages 289 - 294; M.Cope &
D.T.Delpy: "System for long-term measurement
of cerebral blood and tissue oxygenation on
newborn infants by near infra-red
transillumination" see the whole document**
* **APPLIED OPTICS. vol. 30, no. 1, January 1991,
NEW YORK US pages 98 - 105; K.Ono et al.:
"Fiber optic reflectance spectrophotometry
system for in vivo tissue diagnosis" see the
whole document**

## Description

This invention relates to apparatus for the non-invasive quantitative measurement of a substance in a human or animal body, and in particular to apparatus for such measurement utilising electromagnetic radiation.

Whilst apparatus according to the invention may be used in the quantitative measurement of a number of substances in the body it will primarily be described, by way of example, for use in determining a quantitative value for tissue oxygenation.

Adequate oxygenation of tissues of patients in the intensive care unit (ICU) is a fundamental requirement, yet at present there is no routine method of non-invasively monitoring intracellular oxygen in intact tissues. Few technologies exist which have the capability to do this. Non-invasive techniques capable of monitoring oxidative metabolism include Magnetic Resonance Spectroscopy ($^{31}$P MRS); Positron emission tomography (PET); NADH fluorimetry; Somatosensory evoked potentials (CNS only); optical monitoring, e.g. visible spectroscopy, Near Infra Red (NIR) multi-wavelength spectroscopy.

Primary objectives in both adult and neonatal intensive care include prevention of brain injury and maintenance of normal neurological function. Most current monitoring techniques in both of these areas determine oxygenation at sites distant from the brain and do not directly access either cerebral oxygen delivery or oxygen utilisation of the brain.

There is, therefore, a real need for a reliable, safe and continuous method of monitoring the oxygen available at the cellular level for respiration in the brain (and other organs) during critical care situations.

Hypoxia (or hypoxaemia), which is the absence of sufficient oxygen in tissues and blood, is the major cause of anaesthetic-related deaths and is also symptomatic of a number of naturally occurring and technically induced health problems and disorders. Damage resulting from a hypoxic state can occur in a matter of seconds and is often irreversible. Intracellular hypoxia causes diverse physiological responses that depend on the sensitivity of different organ systems to oxygen deprivation.

In normal tissues the continuous delivery of oxygen closely matches the oxidative metabolic requirements of the tissue. These requirements are determined locally and met primarily by regional increases in blood flow and oxygen extraction. Thus, when functional activity is high, oxygen delivery and extraction increase to keep pace with metabolic demand. Similar responses occur in hypoxia when tissues maintain oxygen uptake by maximising blood flow and oxygen extraction.

At present, systemic measurement of oxygen delivery and uptake are used to draw inferences about the availability of oxygen for intracellular processes. These systemic parameters can be helpful when the total supply of oxygen for the body becomes limited; however, they are unsuitable when various tissues respond and adapt differently to changes in regional oxygenation and metabolism.

Thus, any instrument capable of providing continuous, real time, quantitative information on cerebral oxidative metabolism and haemoglobin oxygenation would have significant advantages over current monitoring capabilities.

Living organisms require a continual import of free energy for three major purposes; the performance of mechanical work in muscle contraction and other cellular movements, the active transport of molecules and ions, and the synthesis of macro-molecules and other biomolecules from simple precursors.

Essentially all of the energy needed for cellular metabolism is provided by /glucose. Each molecule of glucose provides the energy to form many molecules of ATP (adenosine triphosphate). This occurs by means of a series of electron transfer reactions in which the hydrogen atoms from the glucose are catalytically combined with the oxygen present in cells, to form water. This process occurs in the mitochondria with the flavoprotein-cytochrome chain of enzymes responsible for the transfer of electrons to oxygen. Each enzyme in the chain is reduced and then re-oxidized as the electron is passed down the line. The enzyme complex, cytochrome $\underline{c}$ oxidase (abbreviated cyt $\underline{aa}_3$) is the terminal member of the mitochondrial respiratory chain and catalyses approximately 95% of all oxygen utilisation in the human body. In the parallel process of oxidative phosphorylation, free energy is conserved in the form of high energy phosphate bonds and stored primarily as ATP and creatinine phosphate. The mechanism is chemiosmotic and involves the transfer of protons across an insulating membrane (the inner membrane that forms the cristae of the mitochondria) the transfer being driven by oxidation in the respiratory chain. (Oxidation is the combination of substance with oxygen, or loss of hydrogen, or loss of electrons; the corresponding reverse processes are called reduction.) Cyt $\underline{aa}_3$ is, therefore, central to cell metabolism. Cyt $\underline{aa}_3$ is present in measurable quantities in the cerebral cortex and other tissue.

When oxygen is unavailable to cyt $\underline{aa}_3$ the enzyme is reduced, the rate of electron transport slows, and oxidative phosphorylation decreases. Therefore, the redox state of cyt $\underline{aa}_3$ is an important indicator of energy provision during pathologic states characterised by disordered oxygen delivery and utilisation. Thus, an ability to continuously measure and monitor the redox state of this oxygen-utilising enzyme in vivo would provide decisive information on the parameter of oxygen sufficiency in tissue(s) or organ(s) in question.

As is well known, radiation in the near infrared region, having wavelengths in the range 700-1300nm, can penetrate soft tissue and bone surrounding a living organ, and the emerging light can be related to oxidative metabolism. In addition, and of significant importance, it is further known that cyt $\underline{aa}_3$ in living body tissue exhibits an oxygen-dependent absorption band in the 700 to 1300nm wavelength range.

When this key enzyme in oxidative reactions is in the presence of sufficient oxygen, a weak absorption band exists in the 780 to 870nm region with a maximum at a wavelength of about 820 to 840nm. The absence of oxygen results in a complete reduction of the enzyme and the disappearance of the absorption band.

British Patent Specification 2075668 discloses apparatus for providing information regarding the oxygenation of specific tissue or organs (e.g. the brain), by monitoring the absorption by cyt $\underline{aa}_3$, of NIR radiation having wavelengths in the abovementioned region.

Haemoglobin also absorbs light in the near infrared region of the spectrum. In addition, haemoglobin absorbs differently depending on whether it is present in its oxygenated form ($HbO_2$) or reduced form (Hb). Thus the optical signals are affected by the amounts of arterial and venous blood in the field of observation. To obtain the cyt $\underline{aa}_3$ signal it is therefore necessary to determine, and remove, the Hb and $HbO_2$ contributions to light absorption in the NIR, and eliminate their interference with the cyt $\underline{aa}_3$ signal. To do this multiple monochromatic light sources are required. Such light sources, together with suitable algorithms, enable simultaneous equations to be constructed and solved for the three unknowns (Hb, $HbO_2$, cyt $\underline{aa}_3$) giving qualitative information about these compounds.

Since three overlapping absorption spectra must be de-convoluted, absorption data are needed for a minimum of three NIR wavelengths to measure contributions by the three molecular species of interest. (Four wavelength algorithms provide more accurate descriptions of NIR absorption and scattering by tissues).

Such apparatus is useful as a trend monitor but quantitative results are unattainable since calibration of the apparatus is not possible for the following reasons:

a) Material such as skin or bone through which the radiation is passing will reduce the radiation intensity both before and after passing through the particular tissue of interest; this will vary from patient to patient.
b) The efficiency with which the incident radiation is guided into the body is unknown and variable, as is the efficiency with which the radiation is transferred from the body to the detector.
c) The path length of the radiation within the tissue under test cannot be accurately determined and can only be estimated by photon time of flight measurements.

As is well known in the art, the path length is critical to the intensity of radiation detected by the detector. This relationship is given by the Beer-Lambert Law.

$$\ln(I_0/I) = d \times E \times c$$

where $\ln = 2.303 \log_{10}$

$I_0$ = Intensity of source radiation impinging on the sample

$I$ = Intensity of radiation transmitted through the sample

$E$ = Absorption (extinction) coefficient of the solute species at the wavelength of the source radiation impinged on the sample

$d$ = Optical distance or path length (travel path length of radiation transmitted through sample)

$c$ = Concentration of substance being measured.

These uncertainties and variables mean that the use of apparatus of the type described above is not a quantitative technique, that is it cannot be applied from patient to patient without calibrating the instrument for each individual patient.

US-A-4 321 930 describes apparatus for non-invasive monitoring of a substance in living tissue comprising an emitter and two detectors. One of the detectors is arranged in close proximity to the emitter so as to sense directly reflected light, the other detector being arranged to sense light which has been scattered and attenuated by the tissue.

According to the invention, there is provided apparatus for non-invasive quantitative measurement of a substance in living tissue, as defined by claim 1.

The invention extends to a method of non-invasive, quantitative measurement of a substance in living tissue using such apparatus.

Advantageously, the apparatus is adapted for continuous and/or discontinuous measurement of said substance such that discrete or continuous measurements can be made.

It is preferred that the emitter means is capable of emitting electromagnetic radiation of a wavelength which the substance under investigation in the body is known to absorb. In one embodiment where the substance under investigation is cyt $\underline{aa}_3$ and/or Hb, and/or $HbO_2$, (the concentrations of which are dependent on tissue oxidative metabolism), it is preferred that the emitter means is capable of emitting electromagnetic radiation of a wavelength between 350 and 1600nm, and more preferably in the range 700 to 1300nm.

Advantageously, the emitter means comprises a plurality of independently actuatable sub-emitters. Preferably, at least three sub-emitters are provided, each being advantageously arranged to emit radiation of a discrete wavelength. In a preferred embodiment four sub-emitters are utilised. The radiation sources for the sub-emitters may, for example, be as described in the above-mentioned British Specification 2075668.

It is preferred that at least one of the first and second detection means, more preferably both, are at least part annular detectors. It is particularly preferred that the first and second detection means should be annular detectors arranged concentrically encircling the emitter means.

The radiation detection means and the means for producing the output signal may be combined, for example, as in a photodiode. Alternatively, the means for producing the electrical signal, for example, a photomultiplier, may be remote from the detection means and connected thereto by a fight guide such as an internally reflecting waveguide or the like.

In apparatus according to the invention, at least the emitter means and first and second radiation detector means are preferably provided in a single housing unit, for ease and convenience of use.

Preferably, the means for processing the first and second output signals include signal conditioning and amplification means. Advantageously, the apparatus may incorporate means for administering oxygen to the patient, which means is advantageously operable when the processed value for the concentration of the substance (e.g. cyt $aa_3$) obtained falls below a predetermined minimum value.

It is preferred that means is provided for attachment of the apparatus to the skin, tissue or organ of the patient.

Whilst the apparatus according to the invention has been described in relation to determining the concentration of substances in the body which are related to oxidative metabolism, it will be appreciated that this form of apparatus may be used for determining the concentration of a wide range of substances in the body by the use of suitable wavelength electromagnetic radiation sources.

The invention will now be further described, by way of example only, with reference to the accompanying drawings, in which:

Figure 1 is a schematic plan view of apparatus according to the invention;
Figure 2 is a cross-section through the apparatus of Figure 1 in use; and
Figure 3 is a block diagram of a control system used with apparatus according to the invention.

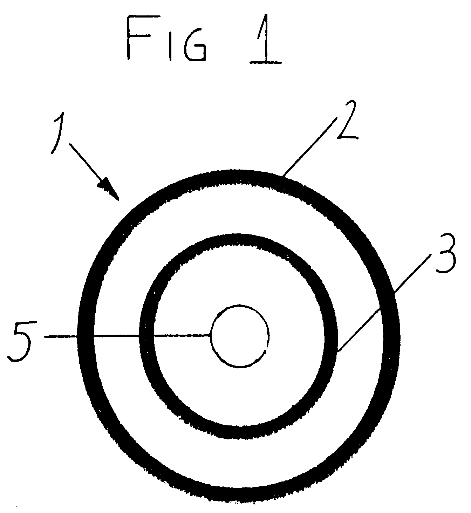

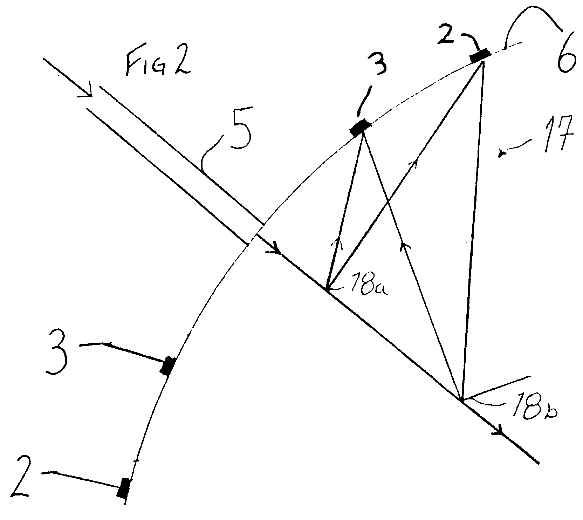

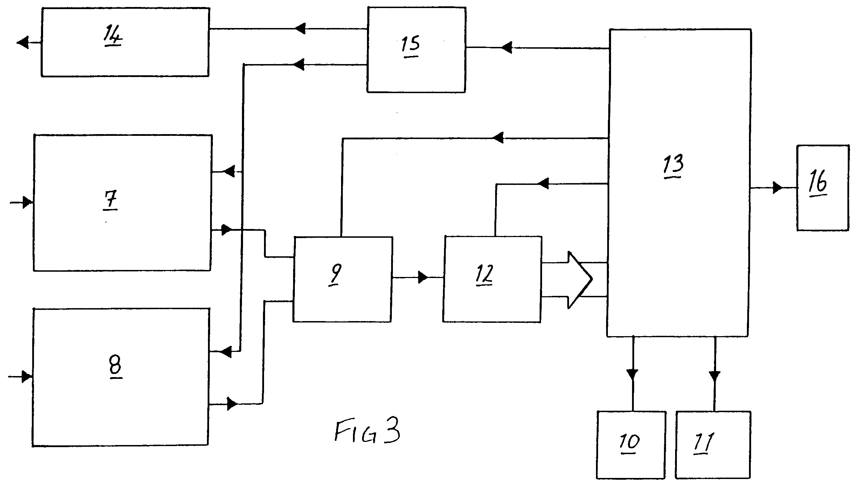

Referring to Figures 1 and 2, a sensor, generally designated 1, consists of a central emitter core 5 carrying light from four sub-emitter laser-diodes each capable of emitting infra-red radiation at a discrete wavelength within the range 700-1300nm. Arranged concentrically around the core 5 is a primary inner annular photodetector ring 3, and spaced therefrom a secondary outer annular photodetector ring 2. Referring to Figure 3, the photodetector rings 2,3 are coupled to respective photomultipliers (not shown), which are in turn connected to respective channels 7,8 of suitable electronic amplification and signal processing circuitry. The output signals from the photodetector rings 2,3 are then passed via signal steering circuitry 9 and analogue to digital convertor 12 to a microprocessor control unit 13 programmed to calculate set algorithms dependent on the ratio of the two output signals enabling a value for the relationship between oxygen availability and oxygen consumption at the cellular level to be obtained, which is then recorded or displayed on a VDU and chart recorder 10 and 11. Data can also be stored on computer 16.

Figure 3 also shows how the laser power supply drive and firing circuitry, 14 and respective signal processing channels 7,8 are both controlled by a timing sequence controller 15 connected to the microprocessor control unit 13.

The calibration of the apparatus enables a value for the relationship between - - oxygen availability and oxygen consumption at the cellular level to be found.

The use of a two detector system enables a reproducible relationship to be established between the path length of the two optical paths used. This relationship will apply from patient to patient, and in this way the Beer-Lambert law can be applied to quantify the substance (in this case e.g. cyt $aa_3$) in the volume of tissue 17 under investigation beneath the sensor.

The assumption is made here that the coupling efficiencies between the body and the two detectors are equivalent and also that the signal attenuation caused by the radiation passing through the regions contiguous with each detector will be equivalent for each detector.

This means that the signal attenuation measured between photodetectors 3 and 2 should be due to absorption occurring in the predicted measurement region.

Referring to Figure 2 in particular, in use the sensor is placed on the tissue surface 6 (e.g. the head) with the annular photodetector rings 2,3 and emitter core 5 in contact therewith, the radiation (in this case near infrared radiation) enters the tissue and scatters (see arbitrary scattering points 18a, 18b) to follow multiple paths around inside the tissue before being detected by the annular photodetector rings 2,3. The signal generated at the photodetector rings is then amplified and processed as described above.

The apparatus according to the invention provides further advantages in the following areas:

1. The arrangement of the photodetectors 2,3 concentrically around the emitter core 5 allows very large area detectors, while still maintaining constant light path lengths to all parts of each detector. This increases the measured signal strength, without compromising path length considerations, and averages out local irregularities in skin pigmentation (e.g. freckles).
2. Due to its unitary construction, positioning of the sensor requires less skill, and has less effect on the results obtained that in the case of the separate emitter-detector arrangement.
3. The reduced sensitivity to the positioning of the sensor should mean that the sensor is less prone to motion artefact, a factor of great importance in any optical system.

## Claims

1. Apparatus for non-invasive monitoring of a substance in living tissue, which apparatus comprises:

   a) emitter means (5) capable of emitting electromagnetic radiation, said emitter means (5) being arrangeable in use in contact with the skin, tissue or organ of a patient,
   b) first radiation detection means (3) spaced from said emitter means, and arrangeable in use in contact with the skin, tissue or organ of said patient,
   c) means for producing a first electrical output signal dependent on the intensity of the radiation detected by said first radiation detection means,
   d) second radiation detection means (2) spaced from said emitter means (5) by a distance greater than said spacing between said first radiation detection means (3) and said emitter means (5), and arrangeable in use in contact with the skin, tissue or organ of said patient,
   e) means for producing a second electrical output signal dependent on the intensity of the radiation detected by said second radiation detection means (2), characterised in that said first and second radiation detection means (3, 2) being arranged to detect radiation which has been scattered and attenuated by said skin, tissue or organ of said patient and in that said apparatus further comprises signal processing means (12, 13) arranged to provide a quantitative measure of said substance in said living tissue dependent on the ratio of said first and second output signals.

2. Apparatus according to claim 1, wherein said emitter means (5) is capable of emitting electromagnetic radiation of a characteristic absorption wavelength for said substance.

3. Apparatus according to claim 1 or claim 2, wherein said emitter means (5) is capable of emitting electromagnetic radiation of a wavelength between 700 and 1300 nm.

4. Apparatus according to any preceding claim, wherein said emitter means (5) comprises a plurality of independently actuatable sub-emitters.

5. Apparatus according to claim 4, wherein each sub-emitter is arranged to emit radiation of a discrete wavelength.

6. Apparatus according to claim 4 or claim 5, which comprises four said sub-emitters.

7. Apparatus according to any preceding claim, wherein at least one of the first and second detection means (3, 2) is at least part annular.

8. Apparatus according to any preceding claim, wherein said first and second detection means (3, 2) comprise annular detectors arranged concentrically around the emitter means (5).

9. Apparatus according to any preceding claim, wherein the radiation detection means (3, 2) and the means for producing the output signal are combined.

10. Apparatus according to any preceding claim, wherein at least the emitter means (5) and first and second radiation detector means (3, 2) are provided in a unitary housing.

11. Apparatus according to any preceding claim, which apparatus further comprises means for administering oxygen to a patient.

12. Apparatus according to any precling claim, which includes means for attachment of the apparatus to the skin of the patient.

13. A method of non-invasive quantitative measurement of a substance in living tissue using apparatus according to any preceding claim.

## Patentansprüche

1. Gerät zur nichtinvasiven Überwachung einer Substanz in lebendem Gewebe, welches umfaßt:

   a) Emittermittel (5), die elektromagnetische Strahlung aussenden können, wobei die Emittermittel (5) bei Benutzung in Kontakt mit der Haut, dem Gewebe oder Organ eines Patienten angeordnet werden können,
   b) erste Strahlungsnachweismittel (3), die sich in einem Abstand vom Emitter befinden, und bei Benutzung in Kontakt mit der Haut, dem Gewebe oder Organ des Patienten angeordnet werden können,
   c) Mittel zum Erzeugen eines ersten elektrischen Ausgangssignals, das von der Intensität der durch die ersten Nachweismittel erfaßten Strahlung abhängig ist;
   d) zweite Strahlungsnachweismittel (2), die sich in einem Abstand von den Emittermitteln (5) befinden, der größer ist als der Zwischenraum zwischen den ersten Strahlungsnachweismitteln (3) und den Emittermitteln (5), und die bei Benutzung in Kontakt mit der Haut, dem Gewebe oder Organ des Patienten angeordnet werden können;

e) Mittel zum Erzeugen eines zweiten elektrischen Ausgangssignals, das von der Intensität der durch die zweiten Nachweismittel (2) erfaßten Strahlung abhängig ist, dadurch gekennzeichnet, daß die ersten und zweiten Strahlungsnachweismittel (3, 2), die zum Erfassen von Strahlung angeordnet sind, welche durch die Haut, das Gewebe oder Organ des Patienten gestreut und gedämpft wurde, und daß das Gerät ferner Signalverarbeitungsmittel (12, 13) enthält, die angeordnet sind, um eine quantitative Messung der Substanz im lebenden Gewebe zu liefern, die von dem Verhältnis der ersten und zweiten Ausgangssignale abhängt.

2. Gerät nach Anspruch 1, bei dem die Emittermittel (5) elektromagnetische Strahlung mit einer charakteristischen Absorbtionswellenlänge für die Substanz aussenden können.

3. Gerät nach Anspruch 1 oder 2, bei dem die Emittermittel (5) elektromagnetische Strahlung einer Wellenlänge zwischen 700 und 1300 nm aussenden können.

4. Gerät nach einem der vorhergehenden Anspüche, bei dem die Emittermittel (5) eine Vielzahl von selbständig steuerbaren Nebenemittern enthält.

5. Gerät nach Anspruch 4, bei dem jeder Nebenemitter angeordnet ist, um eine Strahlung einer diskreten Wellenlänge auszusenden.

6. Gerät nach Anspruch 4 oder 5, welches vier Nebenemitter enthält.

7. Gerät nach einem der vorhergehenden Ansprüche, bei dem mindestens eines, das erste oder zweite Nachweismittel (3, 2) mindestens zum Teil ringförmig ist.

8. Gerät nach einem der vorhergehenden Ansprüche, bei dem das erste und zweite Nachweismittel (3, 2) ringförmige Detektoren enthält, die konzentrisch um die Emittermittel (5) angeordnet sind.

9. Gerät nach einem der vorhergehenden Ansprüche, bei dem die Strahlungsnachweismittel (3, 2) und die Mittel zum Erzeugen des Ausgangssignals kombiniert sind.

10. Gerät nach einem der vorhergehenden Ansprüche, bei dem mindestens die Emittermittel (5) und die ersten und zweiten Strahlungsnachweismittel (3, 2) in einem einheitlichen Gehäuse bereitgestellt werden.

11. Gerät nach einem der vorhergehenden Ansprüche, wobei das Gerät ferner Mittel zum Verabreichen von Sauerstoff an den Patienten enthält.

12. Gerät nach einem der vorhergehenden Ansprüche, welches Mittel zum Anbringen des Geräte auf die Haut des Patienten enthält.

13. Verfahren zum nichtinvasiven quantitativen Messen einer Substanz in einem lebenden Gewebe unter Verwendung eines Gerätes entsprechend einem der vorhergehenden Ansprüche.

**Revendications**

1. Appareil pour la surveillance non-invasive d'une substance dans un tissu vivant, lequel appareil comprend:

   a) des moyens d'émission (5), capables d'émettre un rayonnement électromagnétique, lesdits moyens d'émission (5) pouvant être agencés, pour l'utilisation, en contact avec la peau, un tissu ou un organe d'un patient,
   b) des premiers moyens de détection de rayonnement (3), séparés desdits moyens d'émetteur, et pouvant être agencés, pour l'utilisation, en contact avec la peau, un tissu ou un organe dudit patient,
   c) des moyens pour produire un premier signal électrique de sortie, dépendant de l'intensité du rayonnement détecté par lesdits premiers moyens de détection de rayonnement,
   d) des seconds moyens de détection ge rayonnement (2), séparés desdits moyens d'émission (5) d'une distance supérieure audit espacement entre lesdits premiers moyens de détection de rayonnement (3) et lesdits moyens d'émission (5), et pouvant être agencés, pour l'utilisation, en contact avec la peau, un tissu ou un organe dudit patient,
   e) des moyens pour produire un second signal électrique de sortie, dépendant de l'intensité du rayonnement détecté par lesdits seconds moyens de détection de rayonnement (2),

   **caractérisé en ce que**
   lesdits premiers et seconds moyens de détection de rayonnement (3, 2) sont agencés pour détecter un rayonnement qui a été dispersé et atténué par lesdits peau, tissu ou organe dudit patient, et en ce que ledit appareil comprend en outre
   des moyens de traitement de signal (12, 13), agencés de façon à fournir une mesure quantitative de ladite substance présente dans ledit tissu vivant, en fonction du rapport desdits premier et second signaux de sortie.

2. Appareil selon la revendication 1, dans lequel lesdits moyens d'émetteur (5) sont capables d'émettre un rayonnement électromagnétique d'une longueur d'onde d'absorption caractéristique pour ladite substance.

3. Appareil selon la revendication 1 ou la revendication 2, dans lequel lesdits moyens d'émetteur (5) sont capables d'émettre un rayonnement électromagnétique d'une longueur d'onde comprise entre 700 et 1300 nm.

4. Appareil selon l'une quelconque des revendications précédentes, dans lequel lesdits moyens d'émission (5) comprennent une pluralité de sous-émetteurs pouvant être actionnés indépendamment.

5. Appareil selon la revendication 4, dans lequel chaque sous-émetteur est agencé pour émettre un rayonnement d'une longueur d'onde discrète.

6. Appareil selon la revendication 4 ou la revendication 5, qui comprend quatre sous-émetteurs.

7. Appareil selon l'une quelconque des revendications précédentes, dans lequel au moins l'un des premiers et seconds moyens de détection (3, 2) est au moins partiellement annulaire.

8. Appareil selon l'une quelconque des revendications précédentes, dans lequel lesdits premiers et seconds moyens de détection (3, 2) comprennent des détecteurs annulaires agencés de manière concentrique autour des moyens d'émission (5).

9. Appareil selon l'une quelconque des revendications précédentes, dans lequel les moyens de détection de rayonnement (3, 2) et les moyens pour produire le signal de sortie sont associés.

10. Appareil selon l'une quelconque des revendications précédentes, dans lequel au moins les moyens d'émission (5) et les premiers et seconds moyens de détection de rayonnement (3, 2) sont fournis dans un logement unitaire.

11. Appareil selon l'une quelconque des revendications précédentes, lequel appareil comprenant en outre des moyens pour administrer de l'oxygène à un patient.

12. Appareil selon l'une quelconque des revendications précédentes, qui comporte des moyens pour la fixation de l'appareil à la peau du patient.

13. Procédé de mesure quantitative non-invasive d'une substance dans un tissu vivant, utilisant l'appareil selon l'une quelconque des revendications précédentes.

FIG 1

FIG 2

Fig 3